# EUROPEAN PATENT APPLICATION

(11) **EP 1 433 413 A2**
(43) Date of publication of application: **30.06.2004**
(21) Application number: 03078524.0
(22) Date of filing: 12.03.2002
(51) Int. Cl.: A61B 1/267, A61B 1/00

(54) **Laryngoscope**

(30) Priority: 14.03.2001 AU pr372501; 06.11.2001 AU pr869601
(62) Divisional of application: 02704473.4
(71) Applicant: Western Sydney Area Health Service, Westmead, NSW 2145 (AU); Techmin Pty Ltd, North Rocks, NSW 2151 (AU)
(72) Inventor: Dey, Philip, Kingswood, NSW 2747 (AU); Klineberg, Peter, Castle Hill, NSW 2154 (AU); Stokan, Murray, Winston Hills, NSW 2153 (AU)
(74) Representative: Flynn, Michael Joseph

(57) **Abstract**

An endoscope being a laryngoscope (40) having a handle (42) and a disposable blade (41) characterised in that a light source (46), preferably a light emitting diode (LED), is mounted to the handle of the laryngoscope and the blade has a light transfer conduit (47) adapted to transfer light emitted by the LED from its position on the handle along at least a portion of the blade to one or more light radiating devices (48) on the blade there being a switch to ensure that the LED remains illuminated while the blade is attached to the handle but that the LED is extinguished upon removal of the blade from handle; the switch utilising an induction coil mounted on the handle and a coil or other component capable of carrying an induced electrical current on the blade; the induction coil on the handle and the coil or other component on the blade interacting such that modification in current flowing through the coil on the handle caused by proximity of the induction coil or other component on the blade is detected by circuitry (51) in the handle which modifies the on off state of the LED; none of the components of the switch having exposed contacts or moving parts external to the handle.

## Description

### Field of the Invention

The present invention relates to a pressure sensor and in particular to a pressure sensor for detecting pressure applied to a patient's teeth during use of a laryngoscope. The present invention also relates to a laryngoscope having a light source mounted thereon.

### Background of the Invention

Laryngoscopes are used by physicians, in particular anaesthetists, to perform laryngoscopy and visualise the larynx. Once in place, the anaesthetist can more readily insert endotracheal tubes and the like into the trachea of the patient. The design of laryngoscopes has been relatively unchanged for many decades, with the scope normally comprising a handle and a detachably mounted hook-on blade which are connected together in a substantially L-shaped configuration.

Dental trauma during laryngoscopy is a relatively common complication. Such dental trauma normally results from excess pressure being applied to the upper front teeth of the patient, which can act as a fulcrum.

While leaming laryngoscopy it is difficult for the trainee and supervisor to estimate how much pressure is being applied to the maxillary incisors. During difficult intubations, even experienced laryngoscopists can apply excessive force.

While larynoscopes having pressure sensors have been described in the patent literature (eg. US 5536245), such sensors have not been seen in use by the present inventors. This is postulated by the present inventors to be due to complications in the manufacture and/or use of hitherto known designs.

The present application is directed to a pressure sensor that can be used with or on a laryngoscope that addresses the perceived complications in the art.

During visual examination, a light bulb mounted on the scope can be illuminated to assist in illuminating the area being examined by the anaesthetist or surgeon during use. Such bulbs have typically comprised an incandescent bulb drawing power from one or more batteries mounted in the handle of the scope.

Due to concerns raised by the possibility of cross-contamination arising from the use of larnygoscopes on different patients, larynosoope blades are now routinely sterilised following use on a single patient. Lamygoscope handles are also routinely decontaminated by being wiped with a bactericidal solution. This requirement has significantly increased the stock of laryngoscope blades and handles that must be held in store by any one hospital. Following repeated sterilisations, the performance of the laryngoscope also decreases eventually to the point where it must be discarded. Enquiries by the present inventors have determined that light bulbs mounted on lamyngoscope do not typically last more than three to five sterilisations of the device and must, therefore, be routinely replaced. This requirement to purchase, sterilise, store and continually replace light bulbs on laryngoscopes represents a significant cost for a busy hospital or other medical facility.

The present application is directed to a laryngoscope that addresses the perceived complications in the art.

Any discussion of documents, acts, materials, devices, articles or the like which has been included in the present specification is solely for the purpose of providing a context for the present invention. It is not to be taken as an admission that any or all of these matters form part of the prior art base or were common general knowledge in the field relevant to the present invention as it existed in Australia before the priority date of each claim of this application.

### Summary of the Invention

Throughout this specification the word "comprise", or variations such as "comprises" or "comprising", will be understood to imply the inclusion of a stated element, integer or step, or group of elements, integers or steps, but not the exclusion of any other element, integer or step, or group of elements, integers or steps.

According to a first aspect, the present application is directed to a first invention comprising a laryngoscope comprising a blade and handle, a transducer being attached to the blade, the transducer comprising a circuit having a switch means and an indicator means, the switch means comprising a layer of an electrically conductive polymeric material that is deformable into contact with an electrically conductive contact of the circuit, on presence of a predetermined level of incident pressure, to complete the circuit and so activate the indicator means.

In this aspect, the block of electrically conductive polymeric material is preferably formed from a carbon-loaded silicone rubber. The block preferably has an underside having at least one channel formed therein, each of said at least one channel overlaying a respective one of said electrically conductive contact, the channel being deformable on presence of said predetermined incident pressure into contact with said contact.

Each of said at least one contact can comprise a metal track formed on a printed circuit board. The metal tracks can comprise part of the circuit that is closed when the block contacts the track so as to allow power to activate the indicator means.

In this aspect, the indicator means preferably comprises an alarm means actuable on closure of the circuit. The alarm means preferably comprises a visual means and/or an audible means.

The visual means can comprise one or more lights or light emitting diodes (LED). In another embodiment, the visual means can comprise a readout giving a measure of relative or absolute pressure detected by the transducer. The audible means can comprise a buzzer, bell or the like. The frequency and/or volume of the buzzer can vary in response to changes in incident pressure measured by the transducer. For example, the frequency and/or volume of the buzzer can increase in response to increasing pressure.

According to a second aspect, the present invention is directed to a second invention comprising a sensing means comprising a transducer adapted to be mounted to a blade of a laryngoscope, and an indicator means, the transducer comprising at least a layer of a polymeric material that undergoes a change in resistivity in response to incident pressure thereon, the change in resistivity being useable by the indicator means to provide an output at least indicative of the incident pressure.

According to a third aspect, the present invention is directed to a third invention comprising a laryngoscope comprising a blade and handle, a transducer being attached to the blade, and an indicator means, the transducer comprising at least a layer of a polymeric material that undergoes a change in resistivity in response to incident pressure thereon, the change in resistivity being useable by the indicator means to provide an output at least indicative of the incident pressure.

In one embodiment of the above aspects, the transducer can be formed at least in part of a material that permanently deforms on contact with the teeth of a patient. The degree of permanent deformation of the material of the transducer is preferably proportional to the degree of pressure applied to the transducer by the teeth of the patient. In one embodiment, the permanent deformation comprises depression of said material in the region of contact between the transducer and the teeth of the patient. In this embodiment, the depth of a depression is indicative of the degree of pressure applied to the teeth of the patient, with the deeper the depression, the greater the applied pressure.

According to a fourth aspect, the present invention is directed to a fourth invention comprising a sensing means adapted to be attached to a blade of a laryngoscope, and an indicator means adapted to output at least a relative determination of incident pressure detected by the transducer, the transducer being formed at least in part of a material that permanently deforms on contact with the teeth of a patient.

In a preferred embodiment of the fourth aspect, the degree of permanent deformation of said material is proportional to the degree of pressure applied to the transducer by the teeth of the patient. In one embodiment, the permanent deformation comprises depression of said material in the region of contact between the transducer and the teeth of the patient. In this embodiment, the depth of a depression is indicative of the degree of pressure applied to the teeth of the patient, with the deeper the depression, the greater the applied pressure.

In one embodiment of the second and fourth aspects, a plurality of transducers can be packaged together. For example, a plurality of transducers can be mounted by a release adhesive to a common backing layer. When required, a transducer can be peeled from the backing layer, used, and then discarded.

In the second and third aspects, the polymeric material comprising the transducer can be one or more layers of the polymeric material sold under the name Velostat™ by the company 3M™, ie. a carbon impregnated polyolefin. Other materials having equivalent or similar properties can also be utilised. The electrical resistivity of the material is preferably inversely proportional to incident pressure, the incident pressure causing compression in at least a region of the transducer.

The layer of Velostat™ is preferably sandwiched between respective layers of an electrically conductive material. Each sandwich layer is preferably formed from the same material. The sandwich layers can be maintained in a substantially parallel spaced relationship by the layer of Velostat™. The electrically conductive material can be a metal, such as copper sheet. The sandwich layers act as respective electrodes for the transducer. In a preferred embodiment, the sandwich layers can act as the material that permanently deforms on contact with the teeth of a patient.

The transducer can further include a layer of relatively resiliently flexible material mounted to at least one face thereof. This layer can be selected from the group comprising a foam, an elastomeric material and a polymeric material.

The resiliently flexible material layer can have a layer of adhesive on one or both faces. The face of the layer that becomes the inwardly facing layer following mounting of the transducer preferably has a removable backing layer over the adhesive. The backing layer of the adhesive is preferably removed to allow mounting of the transducer to the blade of the laryngoscope.

The transducer further preferably includes a protective layer on at least one face of the transducer. The protective layer is preferably relatively electrically insulating. The layer is preferably transparent to allow viewing of the electrically conductive layer therebeneath. A layer of adhesive can be used to bond the protective layer to the transducer.

Electrically conducting wires are preferably electrically connected to each of the electrodes of the transducer. The wires preferably are used to provide electrical connection between the transducer and the indicator means. The wires are preferably formed from a metallic material, such as copper or aluminium wire. Each of the wires is preferably surrounded by an electrically insulating material for a majority of its length. The electrical insulation is preferably removed where the wire comes into contact with its respective electrode and where it makes electrical connection to the indicator means.

The wires are preferably connected to each of the electrodes using electrically conductive adhesive tape or an electrically conductive adhesive epoxy. Other suitable bonding techniques, including crimping and soldering can be envisaged.

The indicator means preferably comprises an electrical circuit. The transducer is preferably a component of the electrical circuit. The electrical circuit preferably uses a voltage comparator to detect the change in resistance of the transducer in response to incident pressure. Where the transducer has a layer that decreases in electrical resistivity in response to an increase in incident pressure and vice versa, the circuit preferably notes the change in resistance. When the resistance drops to or below a predetermined threshold, the circuit preferably activates an alarm means that is part of the circuit.

The alarm means can comprise a visual means and/or an audible means. The visual means can comprise one or more lights or light emitting diodes (LED). In another embodiment, the visual means can comprise a readout giving a measure of relative or absolute pressure detected by the transducer. The audible means can comprise a buzzer, bell or the like. The frequency and/or volume of the buzzer can vary in response to changes in incident pressure measured by the transducer. For example, the frequency and/or volume of the buzzer can increase in response to increasing pressure.

The electrical circuitry of all of the aspects can be powered by a power source, such as one or more batteries or mains power. The circuitry can include a light or light emitting diode (LED) that indicates the operational status of the circuit (eg. On/Off). This light or LED can be a different colour or multicolour to that used as the visual means.

Where the circuitry relies upon the exceeding of a predetermined threshold, the threshold for activation of the alarm means can be variable and can be set by the user prior to use or even adjusted during use. In certain instances, such as where intubation is being carried out by trainees, the threshold may be set relatively low to ensure that the alarm means, for example, is activated in response to a relatively small incident pressure being applied to the patient's teeth during use. As the user becomes more experienced with the use of a laryngoscope, the threshold can be increased such that the alarm means only operates in instances where relatively excessive pressure is being applied to the teeth of the patient.

In a still further embodiment, the circuit of any of the above aspects can include a memory means to allow recording of pressure data measured by the transducer. The memory means can preferably automatically, or on request, transmit the recorded data to a playback means such as a personal computer, printer or monitor to allow visualisation of the pressure readings over time.

The transducer is preferably not removable from the blade of the laryngoscope. The blade, including the transducer, is further preferably disposable.

The blade with the transducer mounted thereon is preferably packaged in a sterile container following manufacture and is sterile when removed from the package and mounted to the laryngoscope handle.

In a further embodiment of the above aspects, a light source, such as a high intensity light emitting diode (LED) can be mounted to the transducer. The LED can be mounted to the distal end of the transducer and so provide illumination of the larynx during use.

According to a fifth aspect, the present invention is directed to a fifth invention comprising a method of intubating a patient comprising at least the steps of:
using a laryngoscope having a sensing means, according to the above aspects; and
using the indicator means to monitor the pressure applied to the teeth of the patient during use of the laryngoscope.

According to a sixth aspect, the present invention is directed to a sixth invention comprising an endoscope for insertion in a body cavity or orifice and having at least one light source mounted thereon for providing illumination of the cavity or orifice, the endoscope being characterised in that the light source is a light emitting diode.

In the sixth aspect, the endoscope can comprise a laryngoscope. In another embodiment, the endoscope can comprise an otoscope.

In one embodiment, the endoscope is disposable after a single use. In this embodiment, the endoscope is preferably formed from a plastics material.

In the sixth aspect, the laryngoscope can comprise a handle and a blade. The blade can be non-removably attached to the handle at a first end thereof. In another embodiment, the blade is removably attachable to the handle. The blade preferably has a proximal end and a distal end with its proximal end attachable to the handle. The orientation of the blade to the handle can be fixed. Alternatively, the blade orientation can be adjustable.

In one embodiment of the sixth aspect, the light emitting diode (LED) can comprise a gallium arsenide (GaAs) LED. Other suitable light emitting diodes having suitable luminous intensities can be utilised. In one embodiment, the luminous intensity is preferably at least 5600mcd, more preferably at least 6000mcd, and still more preferably at least between 10000 and 15000mcd.

In a further embodiment of the sixth aspect, more than one light source can be mounted to the endoscope.

In one embodiment, the LED can be mounted to the blade of the laryngoscope. More preferably, the LED can be mounted to the handle of the laryngoscope. In this case, the LED is preferably mounted to the handle at or adjacent its first end. The LED is preferably non-removably mounted to the handle.

The blade can include a light transfer means adapted to transfer light emitted by the LED from its position on the handle through at least a portion of the blade. In one embodiment, the light transfer means has a first end at or adjacent the proximal end of the blade. A second end of the light transfer means is positioned on the blade at a location distal the proximal end of the blade. The second end of the light transfer means may be at or adjacent the distal end of the blade or positioned back along the blade at a desired distance from its distal end.

In one embodiment, the light transfer means can comprise a cylindrical member. Members having other suitable shapes can be envisaged. The member is preferably straight, however, non-straight members could be utilised. In one embodiment, the member can be formed from an acrylic material. The light transfer means preferably serves to direct the light emitted from the LED through the blade and out into the body cavity or orifice into which the laryngoscope has been inserted.

In a still further embodiment, the endoscope can incorporate a switching means for use in activating and/or deactivating the light source. In one embodiment, the switching means can be operable by a user of the endoscope.

In a more preferred embodiment of the laryngoscope, the LED is preferably activated when the blade is mounted to the handle. In this case, the LED preferably remains illuminated while the blade is attached to the handle. The LED preferably switches off on removal of the blade from the handle.

In this case, the switching means can comprise an actuable member mounted on the handle that is activated by a complementary actuating member on mounting of the blade to the handle. For example, the handle and blade can have complementary bayonet type fittings to allow the blade to be attached to the handle.

In another embodiment of the sixth aspect, the switching means can utilise an induction coil mounted within the handle. The blade can also incorporate a coil or magnetic component. On attachment of the blade to the handle, the current flowing through the coil in the handle is modified. This modification can be detected by circuitry in the handle and lead to illumination of the light source.

The coil in the handle and the blade, can be comprised of at least two turns of electrically conductive wire. The coil in the handle is preferably tuned to parallel resonance by a capacitor that is part of the circuitry.

The induction coil in the handle can also preferably be used as a means of inductively charging the batteries stored within the handle of the device. A charger can receive the handle and inductively charge the batteries within the handle. Each charger can preferably be used to charge more than one handle.

The inductive coupling between the handle and the coil can also act as a means of transferring signals from the transducer, when present, to the circuit, when the circuit is located within the handle.

The handle can have a cavity for containing the circuitry for operation of the light source as defined above. The power source for the light source and circuitry is also preferably housed within the cavity. The power source preferably comprises one or more batteries. Each battery is preferably non-removable from the handle. The batteries can also be rechargeable to allow re-use of the batteries. The handle is preferably sealed to prevent fluid ingress therein.

According to a seventh aspect, the present invention is directed to a seventh invention comprising a method of intubating a patient comprising the step of using the laryngoscope as defined herein as the sixth aspect of the invention.

### Brief Description of the Drawings

By way of example only, preferred embodiments of the invention are now described with reference to the accompanying drawings, in which:
Fig. 1 is a simplified side elevation view of one embodiment of a transducer according to the present invention;
Fig. 2 is a schematic view of one embodiment of a transducer and indicator means according to the present invention;
Fig. 3 is a depiction of a patient undergoing laryngoscopy using a laryngoscope having a transducer according to the present invention removably attached thereto;
Fig. 4 is a depiction of a patient undergoing laryngoscopy using a laryngoscope having a light emitting diode (LED) mounted thereon;
Fig. 5 is a cut-away perspective view of the handle and blade of the laryngoscope;
Fig. 6 is another cut-away perspective view of the laryngoscope of Fig. 5;
Figs. 7a-7c are various perspective views of the laryngoscope blade; and
Figs 8a and 8b are side elevational and inverse plan views of a block of material for use as part of a switch for another embodiment of a sensing means according to the present invention.

### Preferred Mode of Carrying out the Invention

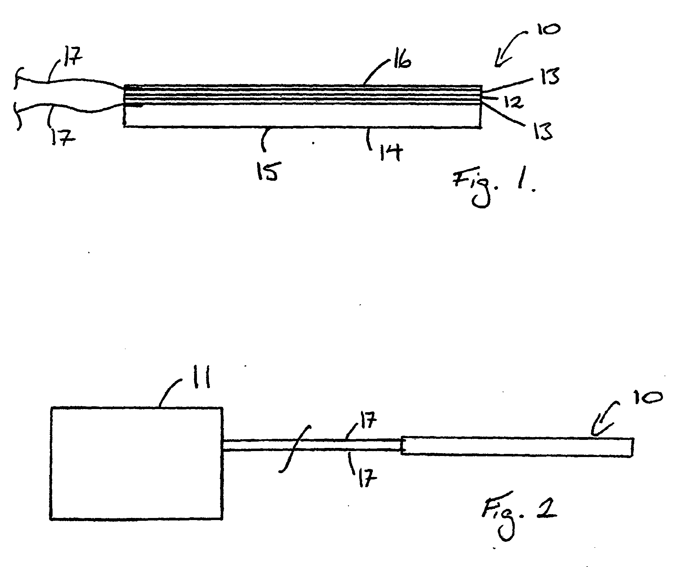

One embodiment of a sensing means having a transducer according to the present invention is generally depicted as 10 in Figs 1 to 3.

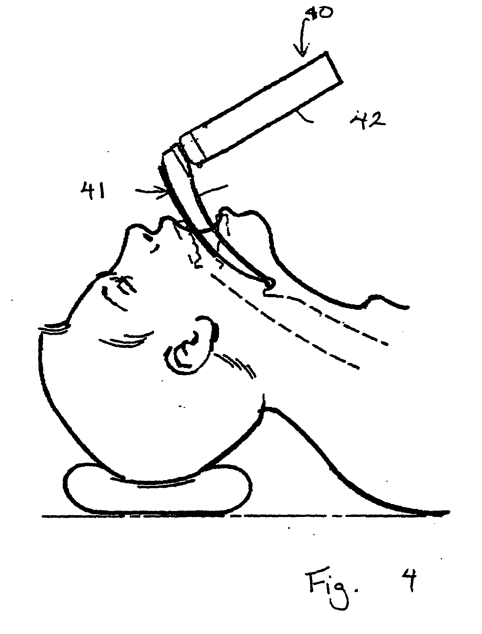

As depicted in Fig. 3, the transducer 10 is adapted to be removably adhered to a blade 41 of a laryngoscope 40. While the depicted transducer can be removably attached to the blade 41, it should be appreciated that a laryngoscope having a non-removable transducer attached thereto is also encompassed within the scope of the present invention.

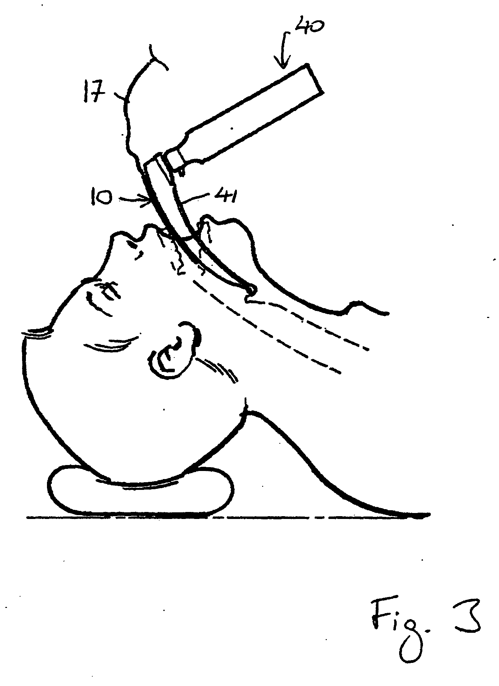

The device further includes an indicator device depicted schematically as 11 in Fig. 2 which is described in more detail below.

In the depicted embodiment, the transducer 10 is formed of a layer of polymeric material 12 sold under the name Velostat™ by the company 3M™, le. a carbon impregnated polyolefin. The electrical resistivity of this layer 12 is inversely proportional to incident pressure, the incident pressure causing compression in at least a region of the transducer 10.

The layer 12 of Velostat™ is sandwiched between respective copper electrodes 13 that are maintained in a substantially parallel spaced relationship by the layer 12 of Velostat™. While copper electrodes are preferred due to the permanent deformation suffered by the material on being brought into contact with the patient's teeth, other electrically conductive materials could be utilised.

As depicted, the transducer 10 can further include a layer 14 of relatively resiliently flexible material mounted to at least one face thereof. Layer 14 can be selected from the group comprising a foam, an elastomeric material and a polymeric material.

The resiliently flexible material layer 14 can have a layer of adhesive on one or both of its faces. The face 15 of the layer 14 that becomes the inwardly facing layer following mounting of the transducer 10 the blade 41 preferably has a removable backing layer (not depicted) over the adhesive.

The transducer 10 further has a protective layer 16 on what becomes its outward face. The depicted protective layer 16 is relatively electrically insulating and transparent to allow viewing of the electrode 13 therebeneath. A layer of adhesive can be used to bond the protective layer 16 to the electrode 13.

The depicted transducer 10 is packaged in a sterile container following manufacture and should be sterile when removed from the package and mounted to the laryngoscope blade 41. A plurality of transducers 10 can be packaged together and delivered ready for individual use. For example, while not depicted, a plurality of transducers 10 can be mounted by a release adhesive to a common backing layer. When required, a transducer can be peeled from the backing layer, used, and then discarded.

Electrically conducting wires 17 are connected to each of the electrodes 13 of the transducer 10. The wires 17 provide electrical connection between the transducer 10 and the indicator device 11.

The depicted wires 17 are connected to each of the electrodes 13 using electrically. conductive adhesive tape or an electrically conductive adhesive epoxy. Other suitable bonding techniques, including crimping and soldering can be envisaged.

The indicator device 11 comprises an electrical circuit. The transducer 10 is a component of this electrical circuit. The depicted indicator device 11 uses a voltage comparator to detect the change in resistance of the transducer 10 in response to incident pressure. As the layer 12 decreases in electrical resistivity in response to an increase in incident pressure and vice versa, the circuit detects the change in resistance. When the resistance drops to or below a predetermined threshold, the circuit activates a buzzer and/or illuminates a light emitting diode mounted in the indicator device 11.

While not depicted, the indicator device can include a readout giving a measure of relative or absolute pressure detected by the transducer 10. The frequency and/or volume of the buzzer varies in response to changes in incident pressure measured by the transducer. In this example, the frequency and/or volume of the buzzer increases in response to increasing pressure.

The electrical circuitry of the depicted indicator device 11 is powered by one or more batteries. The circuitry includes an LED that indicates the operational status of the circuit (eg. On/Off). This LED is a different colour to that described above. The indicator device also includes an On/Off switch that allows a user to connect/disconnect power to the circuitry when desired.

The threshold of the circuitry is variable and can be set by the user prior to use or even adjusted during use. In certain instances, such as where intubation is being carried out by trainees, the threshold may be set relatively low to ensure that the indicator means operates in response to the pressure being applied to the patient's teeth during use. As the user becomes more experienced with the use of a laryngoscope, the threshold can be increased such that it only operates in instances where relatively excessive pressure is being applied to the teeth of the patient.

In use, the transducer 10 will firstly be removed from its sterile packaging and adhered to the blade 41 as depicted in Fig. 3. The wires 17 can then be electrically connected to the indicator device 11.

During use, any pressure applied to the patient's teeth by the blade 41 is detected by the transducer 10. The circuitry within the indicator device 11 can be set such that the LED and/or buzzer of the indicator device 11 only activates when a certain threshold is reached.

Following completion of the laryngoscopy, the blade 41 is removed from the mouth. The transducer 10 can then be peeled from the blade 41 and discarded.

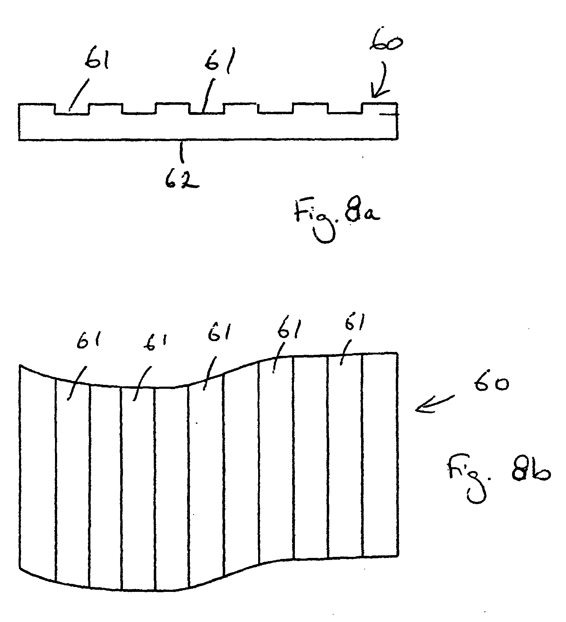

Figs. 8a and 8b depict an alternative component for use as a switch for activating the indicator means 11. The component comprises a block 60 of an extrinsically conductive material, namely a carbon-loaded silicone rubber. The block 60 has a plurality of channals 61 formed in the underside thereof. On the occurrence of an incident pressure on the top side 62 of the block 60, one or more of the channels will deform sufficiently to bring at least a portion of the block into contact with a metal track of a printed circuit board that can pass therebeneath (not depicted).

The block 60 and metal track act together as a switch to control the supply of a power to an indicator means 11 used in association with the switch. When one or more of the channels 61 collapse, the circuit is closed and power is provided to the indicator means 11 so activating an alarm means, such as a buzzer and/or light.

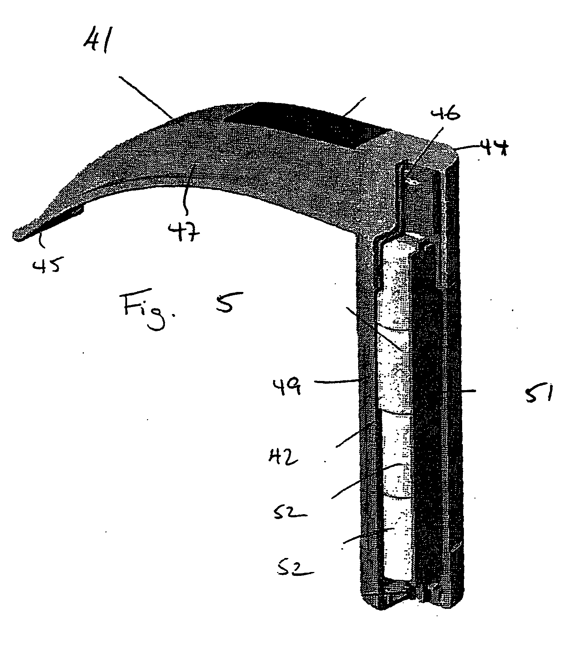

Fig. 4 depicts another embodiment of a laryngoscope according to the present invention that can be used by physicians, in particular anaesthetists, to perform laryngoscopy and visualise the larynx. Once in place, the anaesthetist can more readily insert endotracheal tubes and the like into the trachea of the patient.

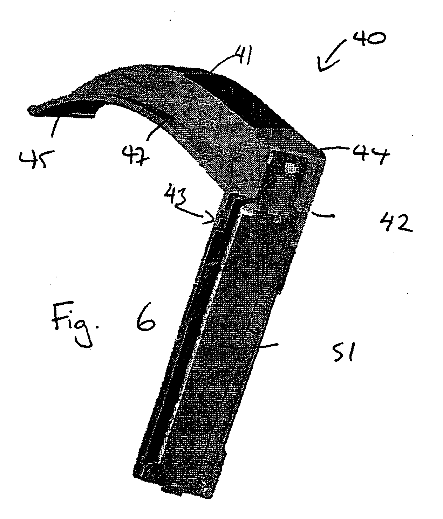

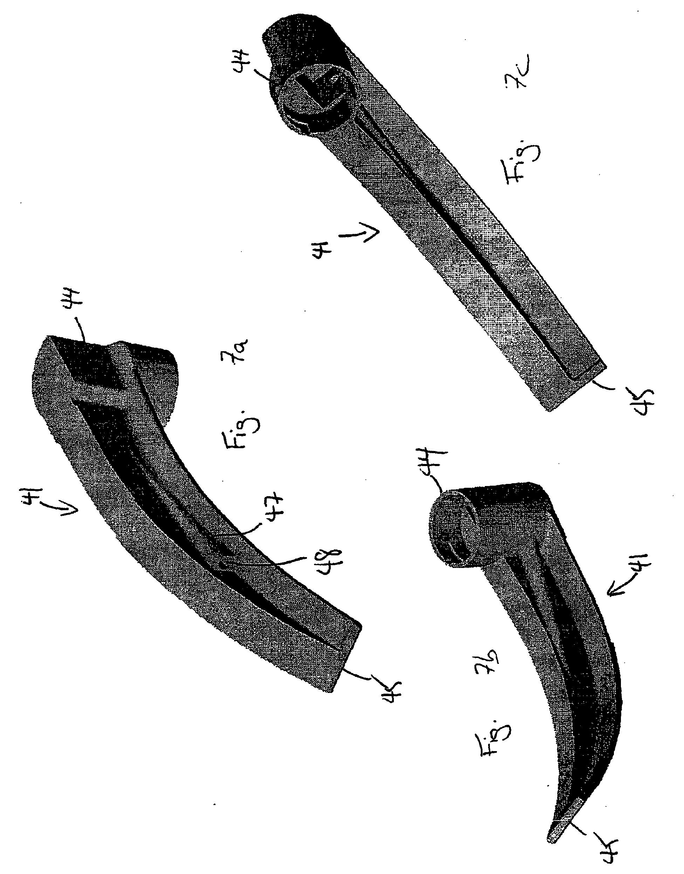

The laryngoscope according to the present invention is generally depicted as 40 in Figs 4-7c.

The laryngoscope 40 depicted in Fig. 4 is disposable and comprises a handle 42 and a blade 41. In this embodiment, both the handle 42 and blade 41 are formed from a plastics material. Suitable materials include polycarbonate, or a copolymer from the ABS (acrylonitrile-butadiene-styrene) family.

The depicted blade 41 is removably attachable to the handle 42 through a bayonet fitting 43. The blade 41 has a proximal end 44 and a distal end 45 with its proximal end 44 attachable to the handle 42. In the depicted embodiment, the orientation of the blade 41 to the handle 42 once attached is fixed. In another embodiment, the blade 41 could be constructed so as to be adjustable relative to the handle 42.

The laryngoscope 40 has a light source 46 mounted thereon for providing illumination of the cavity or orifice, during use. The light source 46 comprises a high intensity gallium arsenide (GaAs) light emitting diode (LED) supplied by Nichia Corporation of Tokushima 774-8601, Japan. Other suitable light emitting diodes having suitable luminous intensities, including LEDs from other suppliers can be utilised.

In the depicted embodiment, the LED 46 is non-removably mounted to the handle 42 of the laryngoscope 40.

The blade 41 has a straight cylindrical acrylic light pipe 47 incorporated therein that transfers light emitted by the LED 46 from its position on the handle 42 through the blade 41 to an outlet 48. The light pipe 47 serves to direct the light emitted from the LED 46 through the blade 41 and out into the body cavity or orifice into which the laryngoscope 40 has been inserted.

The laryngoscope incorporates a switching means for use in activating and/or deactivating the LED 46. In this embodiment, the LED 46 is activated when the blade 41 is mounted to the handle 42 and remains illuminated while ever the blade 41 is attached to the handle 42.

The handle 42 has a cavity 49 for containing the circuitry 51 for operation of the LED 46 as defined above. The power source for the LED and circuitry 51 is also housed within the cavity 49 and comprises a series of batteries 52. The batteries 52 in the depicted embodiment are not removable from the handle 42. They are, however, rechargeable to allow re-use of the handle in association with a blade.

In a typical use, a new blade 41 will be removed from sterile packaging and attached to a handle 42. On attachment, the LED 46 will illuminate and the laryngoscope 40 can be used and positioned by the anaesthetist as depicted in Fig. 4. Following use, the blade 41 can be removed from the handle 42 and disposed of. As, in the depicted embodiment, there is either no or minimal circuitry or wires mounted within the blade 41, the blade 41 is readily disposable at minimum cost. It will be appreciated that in another embodiment, the blade 41 could incorporate other features, including circuitry and other devices, if desired. If necessary, the handle 42 can be sterilised ready for re-use with a new blade 41 when required.

It will be appreciated by persons skilled in the art that numerous variations and/or modifications may be made to the invention as shown in the specific embodiments without departing from the spirit or scope of the invention as broadly described. The present embodiments are, therefore, to be considered in all respects as illustrative and not restrictive.

## Claims

1. An endoscope for insertion in a body cavity or orifice and having at least one light source mounted thereon for providing illumination of the cavity or orifice, the endoscope being **characterised in that** the light source is a light emitting diode.

2. An endoscope in accordance with claim 1 hereof wherein the endoscope is a laryngoscope comprising a handle and a disposable blade, the blade being removably the attached to the handle.

3. An endoscope in accordance with claim 1 hereof wherein the endoscope is a laryngoscope having a handle and a disposable blade; one or more light radiating devices being mounted on the blade of the laryngoscope.

4. An endoscope in accordance with claim 1 hereof wherein the endoscope is a laryngoscope having a handle and a disposable blade **characterised in that** the LED is mounted to the handle of the laryngoscope.

5. An endoscope in accordance with claim 1 hereof wherein the endoscope is a laryngoscope having a handle and a disposable blade **characterised in that** the LED is mounted to the handle of the laryngoscope and the blade has a light transfer conduit adapted to transfer light emitted by the LED from its position on the handle along at least a portion of the blade to one or more light radiating devices on the blade.

6. An endoscope in accordance with claim 1 hereof wherein the endoscope is a laryngoscope having a handle and a disposable blade **characterised in that** the LED is mounted to the handle of the laryngoscope and the blade has a light transfer conduit adapted to transfer light emitted by the LED from a first end at or adjacent a proximal end of the blade to a second end positioned on the blade at a location distal the proximal end of the blade.

7. An endoscope in accordance with claim 1 hereof wherein the endoscope is a laryngoscope having a handle and a disposable blade **characterised in that** the LED is mounted to the handle of the laryngoscope and the blade has an acrylic cylindrical light transfer tube adapted to transfer light emitted by the LED from a first end at or adjacent a proximal end of the blade to one or more light radiating devices at a second end positioned on the blade at a location distal the proximal end of the blade.

8. An endoscope in accordance with claim 1 hereof wherein the endoscope is a laryngoscope comprising a handle and a disposable blade, the blade being removably the attached to the handle and the LED being activated when the blade is mounted to the handle and deactivated when the blade is removed from the handle.

9. An endoscope in accordance with claim 1 hereof wherein the endoscope is a laryngoscope having a handle and a disposable blade **characterised in that** the LED is mounted to the handle of the laryngoscope and the blade has a light transfer conduit adapted to transfer light emitted by the LED from its position on the handle along at least a portion of the blade to one or more light radiating devices on the blade there being a switch to ensure that the LED remains illuminated while the blade is attached to the handle but that the LED is extinguished upon removal of the blade from handle; the switch utilising an induction coil mounted on the handle and a coil or other component capable of carrying an induced electrical current on the blade; the induction coil on the handle and the coil or other component on the blade interacting such that modification in current flowing through the coil on the handle caused by proximity of the induction coil or other component on the blade is detected by circuitry in the handle which modifies the on off state of the LED; none of the components of the switch having exposed contacts or moving parts external to the handle.

10. An endoscope in accordance with claim 1 hereof wherein the endoscope is a laryngoscope having a handle and a disposable blade **characterised in that** the LED is mounted to the handle of the laryngoscope and the blade has a light transfer conduit adapted to transfer light emitted by the LED from its position on the handle along at least a portion of the blade to one or more light radiating devices on the blade there being a switch to ensure that the LED remains illuminated while the blade is attached to the handle but that the LED is extinguished upon removal of the blade from handle; the switch utilising an induction coil mounted on the handle and a coil or other component capable of carrying an induced electrical current on the blade; the induction coil on the handle and the coil or other component on the blade interacting such that modification in current flowing through the coil on the handle caused by proximity of the induction coil or other component on the blade is detected by circuitry in the handle which modifies the on off state of the LED; none of the components of the switch having exposed contacts or moving parts external to the handle; the coil in the handle and the coil in the blade being comprised of at least two turns of electrically conductive wire and the coil in the handle being tuned to parallel resonance by a capacitor.

11. An endoscope in accordance with claim 1 hereof wherein the endoscope is a laryngoscope having a handle and a disposable blade **characterised in that** the LED is mounted to the handle of the laryngoscope and the blade has a light transfer conduit adapted to transfer light emitted by the LED from its position on the handle along at least a portion of the blade to one or more light radiating devices on the blade there being a switch to ensure that the LED remains illuminated while the blade is attached to the handle but that the LED is extinguished upon removal of the blade from handle; the switch utilising an induction coil mounted on the handle and a coil or other component capable of carrying an induced electrical current on the blade; the induction coil on the handle and the coil or other component on the blade interacting such that modification in current flowing through the coil on the handle caused by proximity of the induction coil or other component on the blade is detected by circuitry in the handle which modifies the on off state of the LED; none of the components of the switch having exposed contacts or moving parts external to the handle; the induction coil in the handle being capable of serving the additional function of inductively charging a rechargeable battery stored within the handle of the device when brought into proximity with a complimentary charger adapted to receive the handle.

12. An endoscope in accordance with claim 1 hereof wherein the endoscope is a laryngoscope having a handle and a disposable blade **characterised in that** the LED is mounted to the handle of the laryngoscope and the blade has a light transfer conduit adapted to transfer light emitted by the LED from its position on the handle along at least a portion of the blade to one or more light radiating devices on the blade there being a switch to ensure that the LED remains illuminated while the blade is attached to the handle but that the LED is extinguished upon removal of the blade from handle; the switch utilising an induction coil mounted on the handle and a coil or other component capable of carrying an induced electrical current on the blade; the induction coil on the handle and the coil or other component on the blade interacting such that modification in current flowing through the coil on the handle caused by proximity of the induction coil or other component on the blade is detected by circuitry in the handle which modifies the on off state of the LED; none of the components of the switch having exposed contacts or moving parts external to the handle; a pressure sensing switch on the blade adapted to be activated by excessive pressure between the blade and teeth of a patient; the induction coil in the handle being capable of serving the additional function of transferring signals from the pressure sensing switch on the blade to electronic alarm components in the handle.

13. A method of intubating a patient comprising the step of using an endoscope in accordance with claim 1 hereof.
